# EUROPEAN PATENT SPECIFICATION

(11) **EP 2 792 317 B1**
(45) Date of publication and mention of the grant of the patent: **05.04.2017**
(21) Application number: 14154707.5
(22) Date of filing: 11.02.2014
(51) Int. Cl.: A61B 17/12, A61B 17/29, A61B 17/00, A61B 17/30

(54) **Hemorrhoid ligator**
Hämorrhoidenligator
Ligateur d'hémorroïdes

(30) Priority: 17.04.2013 IT MI20130626
(43) Date of publication of application: 22.10.2014
(73) Proprietor: Sapi Med S.p.A., 15121 Alessandria (IT)
(72) Inventor: Oddenino, Alberto, I-15121 ALESSANDRIA (IT)
(74) Representative: Long, Giorgio

(56) References cited:
- EP-A1- 1 155 660
- WO-A1-2012/151543
- GB-A- 2 426 459
- GB-A- 2 443 605

## Description

The present invention relates to a medical device for applying elastic ligatures to haemorrhoids commonly known as a haemorrhoid ligator.

It is known that haemorrhoids are composed of small venous swellings, so-called haemorrhoidal clusters, which may also come out of the anus and are cause of pain, itching and even bleeding for the patient. An established surgical treatment of haemorrhoids is based on applying an elastic means to the cluster so that the vein is closed at the base and then, being no longer supplied with blood, tends to become sclerotic and fall off spontaneously. Such operation is much simpler and faster than the traditional approach and practically painless and can, in addition, be conducted as an outpatient with local anaesthesia. Such operation, known as ligature of the haemorrhoids is conducted by means of appropriate ligator means constituted essentially of a gun, the rod of which is fitted with suction means. Externally to said barrel a sliding cylinder is placed, actuated by a trigger. The sliding cylinder ends slightly before the distal end of the barrel, thus leaving a portion of the latter free. Suitable elastic means are fitted on such portion for the ligature of the haemorrhoids, generally an elastic ring.

To facilitate the loading of the elastic ring on the free portion of the rod a loading tip or cone is normally used which, after the loading of the elastic rings, is removed. The release of the elastic ring is thus obtained by making the sliding cylinder advance, which thus pushes the elastic ring until it disengages from the rod. The forward movement of the sliding cylinder is operated by a dedicated trigger, positioned near the handle of the instrument, so as to be easy and comfortable to access for the operator.

The elastic ring thus goes to tighten the base of the cluster which was previously sucked inside the aspirator, thus achieving the desired "ligature".

Bearing in mind that a patient normally has various haemorrhoidal clusters, at the end of each ligature the surgeon has to extract the instrument, reconnect the loading cone, load another elastic ring and repeat the operation. It is not in fact practicable to load various elastic rings in one go, in that the poor control which the operator has of the trigger would not permit the safe release of one elastic ring at a time. The repetition of the loading operation of the elastic rings is thus the cause of a significant waste of time for the surgeon and of prolonged discomfort for the patient.

It is in addition evident that for reasons of hygiene and ease of use, such instruments must be disposable. It is therefore extremely important for them to be easy to construct, such as to minimise production costs, and that they can be made of material of limited cost.

Examples of haemorrhoidal ligators of the prior art are described in EP-A-502477, US-A-4257419 and especially in EP-A-1155660 in the Applicant's name.

GB 2443 605 discloses a ligation device comprising an inner member having a ligature mounting portion at one end thereof and an outer member mounted on the inner member. The outer member is mounted on the inner member for relative movement between the members, which movement causes an end of the outer member proximal the ligature mounting portion to pass over the said ligature mounting portion of the inner member.

The purpose of the present invention is therefore to make available a haemorrhoidal ligator which while maintaining the characteristics of ease of construction and limited cost, overcomes the problems described above, making it possible to load various elastic rings on the rod of the instrument in one go.

Said purpose is achieved by a haemorrhoidal ligator as defined in the appended claims, the definitions of which form an integral part of this description.

Further characteristics and advantages of the haemorrhoidal ligator according to the present invention will, in any case, be evident from the description given below of a preferred embodiment, made by way of a nonlimiting example with reference to the appended drawings, wherein:

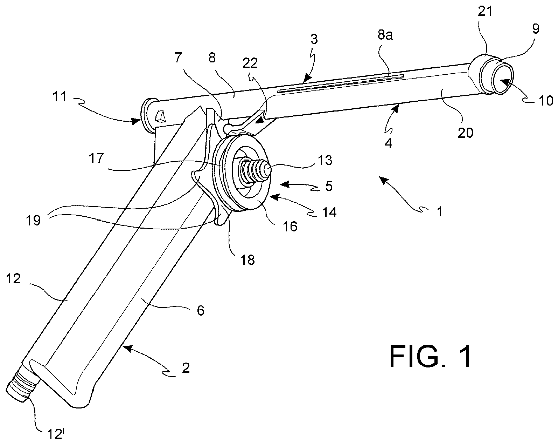

Figure 1 shows a perspective view of a haemorrhoid ligator according to the present invention;
Figure 2A shows a side view of the haemorrhoid ligator in figure 1 in a first operating condition;
Figure 2B shows a side view of the haemorrhoid ligator in figure 2A in a second operating condition;

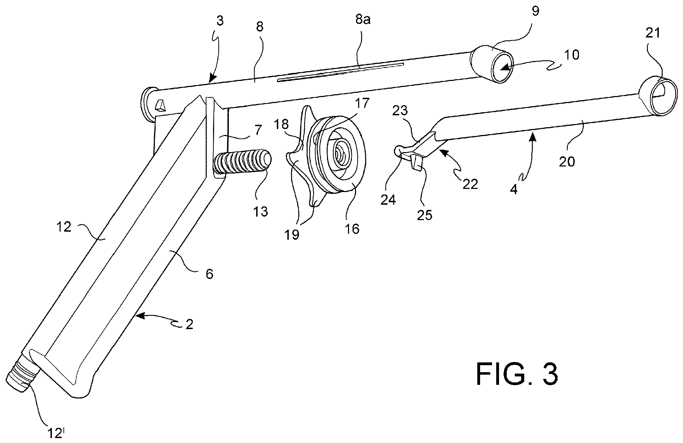

Figure 3 shows an exploded perspective view of the haemorrhoid ligator in figure 1.

With reference to the figures, the haemorrhoid ligator according to the present invention is globally denoted by reference numeral 1. Such ligator comprises a handle 2 fixed to a rod 3 and a slidable member 4 slidably associated to said rod 3. The device further comprises actuating means 5 which act on said slidable member 4.

The rod 3 is composed of a tubular body 8 having the cylindrical distal end 9 inclined downwards in relation to the longitudinal axis of said tubular body and ending with an aperture 10. The inclination of said end 9 is preferably 18°.

A second aperture 11 is placed at the end opposite the tubular body 8.

Reliefs 8 are longitudinally arranged in a symmetric position on the tubular body 8 (in the figures only one relief 8a is visible, the other being positioned on the opposite side of the tubular body 8), acting as guide means for the slidable member 4.

The handle 2 extends along an axis downwardly inclined in relation to the longitudinal axis of the tubular body 8 and comprising a vacuum conduit 12 opening into the core of the tubular body 8 at the coupling point of the handle 2 to the rod 3. The free end of the conduit 12 has a knurled connector 12' for the possible insertion of an elastic tube for the vacuum.

Preferably, the handle 2 and the rod 3 are made in one piece.

The handle 2 comprises a front edge 6 having a connection portion 7 with the rod 3 that is substantially perpendicular to the tubular body 8.

A threaded member 13 extends from the front of said connection portion 7. The threaded member 13 is arranged parallel to the tubular body 8.

The actuating means 5 acting on the slidable member 4 comprise a mobile member 14, which can be screwed onto the threaded member 13 so as to move along the axis X of said threaded member 13 following a manual command.

The mobile member 14 comprises a tubular portion 15, having internally a thread complementary to that of the threaded member 13. A guide member 16 is arranged on the tubular portion 15. The guide member 16 is formed of two facing discs, placed perpendicular to the axis X and defining a circular track 17 which thus unwinds along the rim of the guide member 16.

A control knob 18 is placed on the tubular portion 15 of the mobile member 14 facing the handle 2 and near it. The control knob 18 preferably has a star shape, i.e., it has a central portion from which spokes 19 radiate, in the figure five spokes 19. Preferably the spokes 19 have an arched shape, so as to create, between one spoke and another, an anatomic support for an operator's finger.

The slidable member 4 comprises a body 20 having a semi-cylindrical inner surface. Such body 20 ends at an end with a ring 21 having an inner diameter substantially corresponding to the outer diameter of the distal end 9 of the rod 3. The ring 21 is inclined in relation to the longitudinal axis of the body 20 substantially by the same angle of inclination which the distal end has in relation to the rod 3. Such angle is preferably approximately 18°.

The body 20 of the slidable member 4 has a tab 22 at the end opposite the ring 21. The tab 22 has a first inclined length 23, moving away from the body 20, and a second length 24 substantially parallel to the longitudinal axis of the body 20. A tooth 25 extends downwardly from the second length 24 of the tab 22, which is designed to insert itself in the circular track 17 of the guide member 16.

The haemorrhoid ligator according to the present invention may be made of various materials. It is however preferable for it to be made of plastic, in particular for reasons of cost, preferably being of the disposable type.

It is however useful for the material used, especially as regards the slidable member 4, to be sufficiently elastic. Particularly preferred materials are shock-resistant polystyrene or polycarbonate.

The slidable member 4 is assembled by inserting the ring 21 in the distal end 9 of the rod 3, so that the body 20 is positioned along the tubular body 8 of the rod 3.

The mobile member 14 is inserted by screwing onto the threaded element 13, and the tooth 25 of the tab 22 of the slidable member 4 is housed in the circular track 17 of the guide member 16, thereby forming the actuation means 5.

This way, by making the mobile member 14 rotate, its movement along the threaded element 13 is caused, which could be in one direction rather than the other depending on the direction of rotation given to the control knob 18.

The advancement or retraction of the mobile member 14 causes the simultaneous advancement or retraction of the slidable member 4 on the rod 3 in that it is movable joined to the mobile member 14 by means of the coupling between the tooth 25 and the circular track 17.

The ligator according to the invention is thus used as described below.

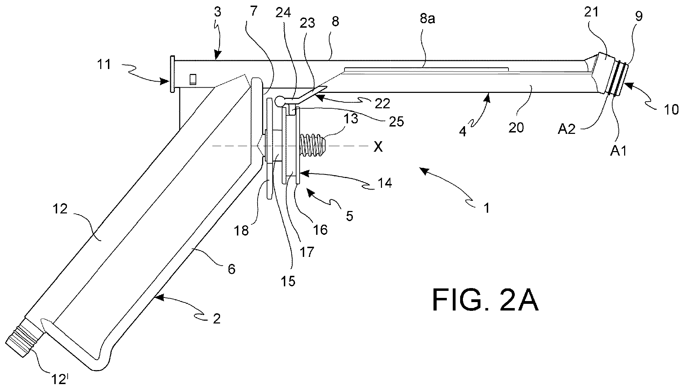

The elastic rings A1, A2 are fitted onto the distal end 9 of the rod 3 as follows. The elastic ring is made to slide along the conical surface of a loading cone (not shown) and is thus increasingly dilated. The elastic ring is thus positioned on the outer surface of the distal end 9 of the rod 3. Using the same procedure further elastic rings may be loaded on the rod 3, as needed, up to a maximum of five or six. Figure 2A shows only two rings A1, A2 by way of example.

The elastic rings are made of a suitable elastomer, which must ensure a significant deformability together with elevated mechanical and chemical resistance properties. A preferred material is natural rubber.

After loading the elastic rings on the distal end 9 of the rod 3, the loading cone is removed.

The device is then connected to vacuum means and inserted in the patient's anus until close to the haemorrhoidal cluster. The instrument is held by the doctor with one hand, exactly like a gun. Using his thumb, the operator closes the aperture 11 of the rod 3 thereby permitting the aspiration of the cluster through the aperture 10 at the opposite end.

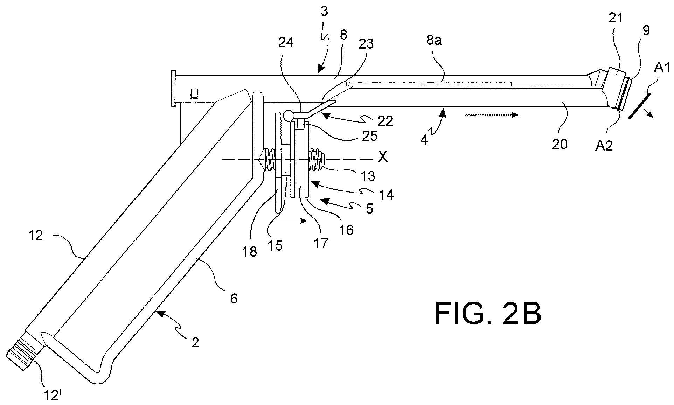

At this point, the operator may rotate the control knob 18, generally using the index or middle finger on the same hand, so as to cause the advancement of the mobile member 14 and thus of the slidable member 4. The ring 21, sliding on the distal end 9 of the rod, pushes a first elastic ring A1 so as to disengage from the rod 3 (figure 2B):

The elastic ring which was fitted forcibly on such distal end of the rod returns to its initial shape, clamping forcibly the base of the cluster. As said above, the cluster, deprived of its blood supply, tends to become sclerotic and drop off.

The operation is repeated on the various haemorrhoidal clusters which the patient suffers from, without the need to reload the ligator with an elastic ring after the ligature of each cluster.

This result is achieved thanks to the fact that compared to the prior trigger systems, the actuation means 5 of the invention permit increased control of the slidable member 4, making it advance in small subsequent steps. With the trigger system of the prior art vice versa, the advancement of the slidable member 4 cannot be limited so as to release one elastic ring at a time.

The advantages of the haemorrhoid ligator according to the present invention are many.

First of all, as said above the device according to the invention makes it possible to add a certain number of elastic rings at the beginning of the operation, preventing the surgeon from having to interrupt after each ligature to reload the instrument.

All this is achieved while keeping production costs low, so that the device according to the invention meets the requirement for a disposable product in the same way as the trigger devices of the prior art.

As said, the haemorrhoid ligator according to the present invention, thanks to its convenience, ease of production and limited cost, is an ideal disposable instrument for the ligature of haemorrhoidal clusters.

It is evident that only some particular embodiments of the haemorrhoid ligator according to the present invention have been described, to which a person skilled in the art may make those modifications required for its adaptation to specific applications, while remaining within the scope of protection as defined in the appended claims.

## Claims

1. An instrument (1) for ligating hemorrhoids, comprising a rod (3) provided with a handle (2) and having an end proximal to the operator and a distal end (9) adapted to receive one or more elastic rings (A1, A2), the distal end (9) and the proximal end of the rod (3) having respective openings (10, 11), wherein the rod is composed of a tubular body (8), and wherein the handle comprises a connection portion (7) with the rod that is substantially perpendicular to the tubular body, wherein the instrument further comprises a slidable member (4) being arranged on the outer surface of said rod (3), which is adapted to disengage upon a command said one or more elastic rings (A1, A2) from said distal end (9) of said rod (3), wherein actuating means (5) of said slidable member (4) are provided, comprising a mobile member (14), which can be screwed on a threaded member (13) associated to said handle (2), so that the mobile member (14) can move along the axis (X) of said threaded member (13) following a manual command, said mobile member (14) being operatively connected with said slidable member (4) for advancing or retracting it wherein the threaded member (13) extends from the front of said connection portion (7) and wherein the mobile member (14) comprises a tubular portion (15), having internally a thread complementary to that of the threaded member (13), a guide member (16) comprising a circular track (17) being arranged on the tubular portion (15), the slidable member (4) comprising a body (20) having a semi-cylindrical inner surface and comprising at a proximal end thereof a tab (22) from which a tooth (25) extends downwardly, said tooth (25) being inserted into the circular track (17) of the guide member (16).

2. The instrument (1) according to claim 1, wherein the mobile member (14) comprises a control knob (18).

3. The instrument (1) according to claim 2, wherein the control knob (18) has a star shape, i.e., it has a central portion from which spokes (19) radiate.

4. The instrument (1) according to claim 3, wherein said spokes (19) have an arched shape, so as to create, between a spoke and the other one, an anatomic support for an operator's finger.

5. The instrument (1) according to any of the claims 1 to 4, wherein the slidable member (4) comprises a ring (21) at the end opposite to the tab (22), the ring (21) having an inner diameter substantially corresponding to the outer diameter of the distal end (9) of the rod (3).

6. The instrument (1) according to claim 5, wherein said ring (21) of the slidable member (4) is slidably associated to the distal end (9) of the rod (3), and both are inclined with respect to the longitudinal axis of the rod (3).

7. The instrument (1) according to claim 6, wherein said ring (21) and said distal end (9) of the rod (3) are inclined by an angle of about 18°.

8. The instrument (1) according to any of the claims 1 to 7, wherein said handle (2) and said rod (3) are in a single piece.

9. The instrument (1) according to any of the claims 1 to 8, wherein said handle (2) comprises a vacuum conduit (12) opening into the rod (3) at the coupling point of the handle (2) to the rod (3).

10. The instrument (1) according to any of the claims 1 to 9, wherein the handle (2) comprises a front edge (6) having a connection portion (7) with the rod (3) that is substantially perpendicular to the rod (3), said threaded member (13) extending from said connection portion (7).

11. The instrument (1) according to any of the claims 1 to 10, wherein the tab (22) has a first inclined length (23), moving away from the body (20), and a second length (24) substantially parallel to the longitudinal axis of the body (20), said tooth (25) extending downwardly from the second length (24).

12. The instrument (1) according to any of the claims 1 to 11, wherein reliefs (8a) are longitudinally arranged in a symmetric position on the rod (3), acting as guide means for the slidable member (4).

## Patentansprüche

1. Instrument (1) zum Ligieren von Hämorrhoiden, umfassend eine Stange (3), welche mit einem Griff (2) bereitgestellt ist und ein zu dem Bediener proximales Ende sowie ein distales Ende (9) aufweist, welches dafür geeignet ist, einen oder mehrere elastische Ringe (A1, A2) aufzunehmen, wobei das distale Ende (9) und das proximale Ende der Stange (3) jeweilige Öffnungen (10, 11) aufweisen, wobei die Stange aus einem rohrförmigen Körper (8) gebildet ist, und wobei der Griff einen Verbindungsabschnitt (7) mit der Stange umfasst, welcher im Wesentlichen lotrecht zu dem rohrförmigen Körper ist, wobei das Instrument ferner umfasst:
ein an der äußeren Fläche der Stange (3) angeordnetes verschiebbares Element (4), welches dafür geeignet ist, auf einen Befehl hin den einen oder die mehreren elastischen Ringe (A1, A2) von dem distalen Ende (9) der Stange (3) zu lösen, wobei Betätigungsmittel (5) des verschiebbaren Elements (4) bereitgestellt sind, welche ein bewegliches Element (14) umfassen, welches auf ein dem Griff (2) zugeordnetes Gewindeelement (13) geschraubt werden kann, so dass sich das bewegliche Element (14) einem manuellen Befehl folgend entlang der Achse (X) des Gewindeelements (13) bewegen kann, wobei das bewegliche Element (14) operativ mit dem verschiebbaren Element (4) verbunden ist, um es vorwärtszubewegen oder einzuziehen, wobei sich das Gewindeelement (13) von der Front des Verbindungsabschnitts (7) erstreckt und wobei das bewegliche Element (14) einen rohrförmigen Abschnitt (15) umfasst, welcher im Inneren ein zu dem des Gewindeelements (13) komplementäres Gewinde aufweist, wobei ein Führungselement (16) eine kreisförmige Spur (17) umfasst, welche an dem rohrförmigen Abschnitt (15) angeordnet ist, wobei das verschiebbare Element (4) einen Körper (20) umfasst, welcher eine halbzylindrische innere Fläche aufweist und an einem proximalen Ende davon eine Lasche (22) umfasst, von welcher sich ein Zahn (25) nach unten erstreckt, wobei der Zahn (25) in die kreisförmige Spur (17) des Führungselements (16) eingefügt ist.

2. Instrument (1) nach Anspruch 1, wobei das bewegliche Element (14) einen Bedienknopf (18) umfasst.

3. Instrument (1) nach Anspruch 2, wobei der Bedienknopf (18) eine Sternform aufweist, d. h. einen zentralen Abschnitt aufweist, von welchem Speichen (19) strahlenförmig wegführen.

4. Instrument (1) nach Anspruch 3, wobei die Speichen (19) eine gebogene Form aufweisen, um zwischen einer Speiche und der anderen eine anatomische Stütze für einen Finger des Bedieners zu erzeugen.

5. Instrument (1) nach einem der Ansprüche 1 bis 4, wobei das verschiebbare Element (4) an dem der Lasche (22) entgegengesetzten Ende einen Ring (21) umfasst, wobei der Ring (21) einen inneren Durchmesser aufweist, welcher im Wesentlichen dem äußeren Durchmesser des distalen Endes (9) der Stange (3) entspricht.

6. Instrument (1) nach Anspruch 5, wobei der Ring (21) des verschiebbaren Elements (4) verschiebbar dem distalen Ende (9) der Stange (3) zugeordnet ist und beide in Bezug auf die Längsachse der Stange (3) geneigt sind.

7. Instrument (1) nach Anspruch 6, wobei der Ring (21) und das distale Ende (9) der Stange (3) um einen Winkel von etwa 18° geneigt sind.

8. Instrument (1) nach einem der Ansprüche 1 bis 7, wobei der Griff (2) und die Stange (3) einstückick ausgebildet sind.

9. Instrument (1) nach einem der Ansprüche 1 bis 8, wobei der Griff (2) eine Vakuumleitung (12) umfasst, welche an dem Verbindungspunkt des Griffs (2) zu der Stange (3) in der Stange (3) mündet.

10. Instrument (1) nach einem der Ansprüche 1 bis 9, wobei der Griff (2) einen vorderen Rand (6) umfasst, welcher einen Verbindungsabschnitt (7) mit der Stange (3) aufweist, welcher im Wesentlichen lotrecht zu der Stange (3) ist, wobei sich das Gewindeelement (13) von dem Verbindungsabschnitt (7) erstreckt.

11. Instrument (1) nach einem der Ansprüche 1 bis 10, wobei die Lasche (22) einen sich von dem Körper (20) wegbewegenden ersten geneigten Längenabschnitt (23) und einen im Wesentlichen zu der Längsachse des Körpers (20) parallelen zweiten Längenabschnitt (24) aufweist, wobei sich der Zahn (25) von dem zweiten Längenabschnitt (24) nach unten erstreckt.

12. Instrument (1) nach einem der Ansprüche 1 bis 11, wobei Aussparungen (8a) longitudinal in einer symmetrischen Position auf der Stange (3) angeordnet sind, welche als Führungsmittel für das verschiebbare Element (4) wirken.

## Revendications

1. Instrument (1) destiné à ligaturer des hémorroïdes, comprenant une tige (3) pourvue d'un manche (2) et ayant une extrémité proximale envers l'opérateur et une extrémité distale (9) adaptée pour recevoir une ou plusieurs bagues élastiques (A1, A2), l'extrémité distale (9) et l'extrémité proximale de la tige (3) ayant des ouvertures (10, 11) respectives, dans lequel la tige est composée d'un corps tubulaire (8), et dans lequel le manche comprend une portion de raccordement (7) avec la tige qui est sensiblement perpendiculaire au corps tubulaire, dans lequel l'instrument comprend en outre un organe coulissant (4) agencé sur la surface externe de ladite tige (3), qui est adapté pour désengager sur ordre lesdites une ou plusieurs bagues élastiques (A1, A2) de ladite extrémité distale (9) de ladite tige (3), dans lequel des moyens d'actionnement (5) dudit organe coulissant (4) sont prévus, comprenant un organe mobile (14), qui peut être vissé sur un organe fileté (13) associé audit manche (2), pour que l'organe mobile (14) puisse se déplacer suivant l'axe (X) dudit organe fileté (13) à la suite d'un ordre manuel, ledit organe mobile (14) étant raccordé opérationnellement audit organe coulissant (4) pour le faire avancer ou rétracter, dans lequel l'organe fileté (13) s'étend depuis l'avant de ladite portion de raccordement (7) et dans lequel l'organe mobile (14) comprend une portion tubulaire (15), ayant à l'intérieur un filet complémentaire de celui de l'organe fileté (13), un organe guide (16) comprenant une voie circulaire (17) qui est agencée sur la portion tubulaire (15), l'organe coulissant (4) comprenant un corps (20) ayant une surface interne semi-cylindrique et comprenant à une extrémité proximale de celle-ci, une patte (22) prolongée vers le bas par une dent (25), ladite dent (25) étant insérée dans la voie circulaire (17) de l'organe guide (16).

2. Instrument (1) selon la revendication 1, dans lequel l'organe mobile (14) comprend une molette de commande (18).

3. Instrument (1) selon la revendication 2, dans lequel la molette de commande (18) a une forme d'étoile, c'est-à-dire, elle comporte une portion centrale de laquelle émergent des rayons (19).

4. Instrument (1) selon la revendication 3, dans lequel lesdits rayons (19) ont une forme arquée, de manière à créer, entre un rayon et l'autre, un support anatomique pour le doigt d'un opérateur.

5. Instrument (1) selon l'une quelconque des revendications 1 à 4, dans lequel l'organe coulissant (4) comprend une bague (21) à l'extrémité opposée à la patte (22), la bague (21) ayant un diamètre interne correspondant sensiblement au diamètre externe de l'extrémité distale (9) de la tige (3).

6. Instrument (1) selon la revendication 5, dans lequel ladite bague (21) de l'organe coulissant (4) est associée en coulissement à l'extrémité distale (9) de la tige (3), et les deux sont inclinées par rapport à l'axe longitudinal de la tige (3).

7. Instrument (1) selon la revendication 6, dans lequel ladite bague (21) et ladite extrémité distale (9) de la tige (3) sont inclinées d'un angle d'environ 18°.

8. Instrument (1) selon l'une quelconque des revendications 1 à 7, dans lequel ledit manche (2) et ladite tige (3) sont d'une seule pièce.

9. Instrument (1) selon l'une quelconque des revendications 1 à 8, dans lequel ledit manche (2) comprend un conduit à vide (12) débouchant dans la tige (3) au niveau du point de couplage du manche (2) à la tige (3).

10. Instrument (1) selon l'une quelconque des revendications 1 à 9, dans lequel le manche (2) comprend un bord avant (6) ayant une portion de raccordement (7) avec la tige (3) qui est sensiblement perpendiculaire à la tige (3), ledit organe fileté (13) prolongeant ladite portion de raccordement (7).

11. Instrument (1) selon l'une quelconque des revendications 1 à 10, dans lequel la patte (22) a une première longueur inclinée (23), s'éloignant du corps (20), et une seconde longueur (24) sensiblement parallèle à l'axe longitudinal du corps (20), ladite dent (25) prolongeant vers le bas la seconde longueur (24).

12. Instrument (1) selon l'une quelconque des revendications 1 à 11, dans lequel des reliefs (8a) sont agencés longitudinalement dans une position symétrique sur la tige (3), servant de moyens guides pour l'organe coulissant (4).
